(19) Europäisches Patentamt
European Patent Office
Office européen des brevets

(11) **EP 4 056 289 B1**

(12) **EUROPEAN PATENT SPECIFICATION**

(45) Date of publication and mention
of the grant of the patent:
**01.05.2024 Bulletin 2024/18**

(21) Application number: **22161228.6**

(22) Date of filing: **10.03.2022**

(51) International Patent Classification (IPC):
**B08B 9/08** *(2006.01)* **B08B 9/093** *(2006.01)*
**B01D 21/24** *(2006.01)*

(52) Cooperative Patent Classification (CPC):
**B08B 9/0933; B01D 21/245;** Y02E 50/30

(54) **METHOD AND SYSTEM FOR REMOVING SAND FROM BIOLOGICAL SLURRY TANKS, AND USE THEREOF IN BIOGAS PRODUCTION PLANTS**

VERFAHREN UND SYSTEM ZUR ENTFERNUNG VON SAND AUS BIOLOGISCHEN GÜLLEBEHÄLTERN UND VERWENDUNG DAVON IN BIOGASERZEUGUNGSANLAGEN

PROCÉDÉ ET SYSTÈME D'ÉLIMINATION DE SABLE DANS DES RÉSERVOIRS DE SUSPENSION BIOLOGIQUE ET LEUR UTILISATION DANS DES INSTALLATIONS DE PRODUCTION DE BIOGAZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2021 DK PA202100254**

(43) Date of publication of application:
**14.09.2022 Bulletin 2022/37**

(73) Proprietor: **Bio Reactor Sand Removing ApS
6800 Varde (DK)**

(72) Inventors:
• **Bech Hermansen, Jonas**
**6830 Nørre Nebel (DK)**
• **Bjerg, Mathias Kristian**
**6830 Nørre Nebel (DK)**
• **Bjerg, Jens Kristian**
**6830 Nørre Nebel (DK)**

(74) Representative: **Andreasen, Søren Laursen
Vasegaard
Tropa ApS
Aagade 97, 1st Floor
8370 Hadsten (DK)**

(56) References cited:
**EP-A1- 1 609 763 EP-A2- 2 497 578
CA-A1- 2 315 670 CA-A1- 2 366 079
US-A- 4 409 746 US-A1- 2011 088 732
US-A1- 2020 222 951**

**Description**

FIELD OF THE INVENTION

The present invention relates to a method and system for cleaning slurry tanks, and use thereof in biogas production plants. In particular, it relates to a method and system according to the preamble of the independent claims.

BACKGROUND OF THE INVENTION

With offset in sand separation from heavy oil, a method is disclosed in Canadian patent CA02366079C in which a lorry with a vacuum collection tank is used to remove sand from a bottom region in an oil storage tank. A manifold with a flexible suction line is attached to an access port at the bottom region of the tank for removing sand by suction into a tank lorry. For stirring up the sand, an injection wand is driven through the manifold and pumping water into the sand, the water being taken from an intermediate region in the oil separation tank. This system has some disadvantages in that the suction line can only suck out sand in the near vicinity of the access port, irrespective of the wand being driven deep into the bottom region.

An improvement relatively to CA02366079C is achieved by the system in CA02315670, disclosing a method in accordance with the preamble of claim 1 and a system in accordance with the preamble of claim 4, where a suction pipe is inserted movably through an access port into the bottom part of an oil separation tank for removal of the sand through the pipe and into a vacuum truck. The movable configuration allows the suction pipe to be inserted deep into the oil separation tank for removal of sand. The pipe comprises an internal tube that injects water into the tank at the front end of the pipe, which stirs up the sand in the vicinity of the front end of the pipe and facilitates removal of the sand by suction through the pipe. Similar to CA02366079C, the water for the injection is taken from the tank itself at a position levelled higher than the access port for the suction pipe, which, however, implies a risk for oil being sucked out of the tank instead of water, which is not working well, may lead to clogging of the pump, and also creates convection in the tank in contrast to the objective of minimizing disturbance. Thus, although, the system in CA02315670 appears useful at first sight, there are some shortcomings in practice.

US20110088732A1 discloses an apparatus for cleaning a tank that is to be installed below the fill line of a tank includes a housing sealably attached to a sidewall of a tank., The apparatus comprises a tubing having a flexible output end positioned within the housing such that the output end of the tubing extends past the sidewall of the tank.

US2020222951 A1 discloses a pipe configured for insertion into a tank to liquefy and remove solids from the tank. The pipe comprises a water injection conduit and a vibrating member configured to impart vibration to the solids. The pipe is designed for removal of produced solids such as sand in the bottom of a heavy oil production storage tank in a cold heavy oil production or steam-assisted gravity drainage production application.

US8734596B1 discloses a siphon adapted for thoroughly cleaning fluid vessels. The siphon is capable of elevating discharge waste fluid through a transfer of kinetic energy provided by a pressurized fluid source, thereby obviating any need for undesirable electrical, chemical, or other mechanical power sources. An inlet couples pressurized fluid to a divider that splits the pressurized fluid between a jet port outlet and a tank flush source conduit. A siphon return conduit is operative to carry waste fluid from the fluid vessel, with the cleaning attachment protruding from the siphon return conduit.

EP2497578A2 discloses an underwater fluid storage tank remediation assembly for removing contaminants from an underwater fluid storage tank. The assembly comprises a flexible pipe which can be inserted into an underwater fluid storage tank and a fluid injection device coupled to the flexible pipe and arranged so that fluid flowing down the pipe passes into the fluid injection device. The fluid injection device comprises at least one fluid outlet for directing a jet of fluid into the tank to agitate contaminants in the tank. The assembly comprises also comprises a pipe gripping and rotation device which can be selectively actuated to grip and rotate the flexible pipe relative to the tank, to facilitate movement of the fluid injection device coupled to the pipe within the tank.

The apparatuses are, however, not suitable for providing a practical and an efficient way of removing sand from a biological slurry tank.

Similar to the oil separation tanks, a tank holding biological slurry for biogas production does also have sand and other particulate debris settle at the bottom of the tank, which have to be removed periodically in order to secure proper continuous functioning. However, in contrast to the methods described above for the oil separation tanks, a different technology is used in biological slurry tanks for biogas production, which is removal of the sand by screw conveyors and cyclones. Although, useful for the purpose, it would be desirable to find a more convenient and easier way to remove sand. Accordingly, there is a need for improvements.

DESCRIPTION / SUMMARY OF THE INVENTION

It is an objective of the invention to provide an improvement in the art. In particular, it is an objective to provide an improvement for biological slurry tanks from which sand has to be removed. This objective is achieved by the method in accordance with claim 1 and the system in accordance with claim 4

As described in the introduction above, the method as described in CA02315670 and CA02366079C requires the slurry tank to be provided with a valve-operated access port at or near the bottom of the tank. This is not always common practice for biological slurry tanks in biogas production plants, and such valve would have to be installed in order to use similar methods.

However, closer testing has revealed that this modification of slurry tanks is not sufficient for applying a method where a pipe is inserted through the access port for removing sand by suction. In particular, when testing the method as described in CA02315670 in relation to slurry tanks, it turned out that the pipe that is inserted through the access port into the tank tends to get stuck due to the stickiness of the material and possible corrosion on the inner wall of such access ports. It appeared that long term storage of biological slurry in a tank appears to have different influence on the access ports than in separation tanks for heavy oil. This is probably due to the fact that oil in tanks prevents substantial corrosion. Accordingly, use of the method and system of CA02315670 for removal of sand from biological slurry tanks in biogas production has turned out not to be immediately applicable. It was found out that further development steps were necessary.

Such further steps were solved after thorough consideration and as explained in the following. By inserting a pipe through an access port of a slurry tank, where the pipe was dimensioned substantially smaller in diameter, for example at least 5% smaller or even at least 10% smaller in diameter, than the free inner diameter of the access port, any corrosion or hardened debris on the walls of the access port would not hinder relatively easy sliding motion of the tube in and out of the access port.

The term "free" inner diameter should be understood as the largest diameter of a circular cylindrical pipe that fits through the access port. This terminology is used because the access port is not necessarily circular, or the flange of the access port may have a different diameter, or there may be other types of obstacles along the inner rim of the access port.

In order to achieve a tight connection between the inserted tube and the access port, so that slurry would not escape from the tank through the space between the pipe and the access port opening when the pipe is inserted, a pipe guide is provided around the pipe and attached to the access port by a liquid-tight connection. Advantageously, the pipe guide is provided with a flange that fits tightly to a corresponding flange of the access port. Thus, although, slurry can flow into the space of the access port around the inserted pipe, the flange connection in combination with the tightening pipe guide prevents further leakage.

The pipe guide has a cylindrical inner wall with an inner pipe guide diameter. The pipe and pipe guide are dimensioned such that the pipe easily slides inside and along the pipe guide but with a tight tolerance so that unwanted leakage of the slurry out of the tank is prevented. For example, the pipe has an outer pipe diameter approximately equal to or slightly smaller than the inner pipe guide diameter so as to prevent leakage between the pipe and the inner wall. In some practical embodiments, sealings are provided between the pipe and the pipe guide. By low-friction sealings, for example made of PTFE (Teflon®), smooth sliding is provided.

As the pipe guide is demounted from the access port after removal of sand from the tank by suction through the pipe, a proper sliding of the pipe inside the pipe guide can easily be maintained by cleaning of the suction pipe equipment, especially the contact region between the pipe and the pipe guide, with or without polymer sealings. This is a simple technical solution for a problem that was characteristic for slurry tanks but not observed for oil tanks, where grease and oil remnants allowed an easy sliding of the pipe through the access port of the oil separation tank despite small tolerances between the outer pipe diameter and the inner free diameter of the access port. And even if the pipe guide is not removed from the access port, removal of the pipe from the pipe guide gives access to the inner volume for cleaning by flushing with water.

The pipe comprises a water conduit inside the pipe for transport of water through the pipe and into the tank to stir-up sand at the front end of the pipe. For example, the water flow is in the form of a jet at the front end of the pipe. The pipe also comprises a suction duct inside the pipe, separate from the water conduit, for transport of stirred-up sand through the pipe in the opposite direction relatively to the water flow.

In one embodiment, the pipe comprises a front portion provided with a cutter head. Hereby, the cutter head is arranged and configured to be used to dredge sediments in the bottom region of a bioreactor. The cutter head comprises a plurality of protruding teeth arranged at the periphery of the cutter head.

In one embodiment, the teeth are integrated in the periphery of the cutter head.

In one embodiment, the cutter head is equipped with receiving structures configured to detachably attach replaceable teeth. Hereby, it is possible to change the teeth after they have worn out in an easy and practical manner. The width of the pipe is equal to or exceeds the width of the cutter head, hereby, it is possible to insert the pipe into the pipe guide.

In accordance with the invention, the pipe is provided with a flow guide arranged and configured to angle the injected water relative to the longitudinal axis of the pipe. The flow guide is arranged to angle the injected water with a predefined angle relative to the longitudinal axis of the pipe. The angle corresponds to the water flow direction of the water leaving the pipe.

In one embodiment, the angle is in the range 10-90 degrees.

In one embodiment, the angle is in the range 10-60 degrees.

In one embodiment, the angle is in the range 25-50 degrees.

In one embodiment, the angle is in the range 20-45 degrees.

In one embodiment, the angle is in the range 60-90 degrees.

In one embodiment, the angle is in the range 75-90 degrees.

The angle may be selected in dependency of the specific application. In some applications the preferred angle in with a range close to 90 degrees such as 75-90 degrees. This would be the case if it is required to inject water towards solid material placed above or below the pipe. In this case it would be possible to inject water towards solid material in order to loosen the solid material.

In one embodiment, the pipe is provided with one or more nozzles arranged and configured to inject water. In one embodiment, the pipe is provided with a plurality of nozzles that are arranged and configured to inject water.

In one embodiment, each nozzle is a rotatably mounted rotation nozzle, wherein the water flow through the nozzle creates rotation of the nozzle, wherein the nozzle is provided at the end distal end of a water supply pipe or a water supply hose.

In one embodiment, the nozzles are arranged and configured to inject a water jet that is angled more than 45 degrees relative to the longitudinal axis of the pipe.

In one embodiment, the nozzles are arranged and configured to inject a water jet that is angled more than 60 degrees relative to the longitudinal axis of the pipe.

In one embodiment, the nozzles are arranged and configured to inject a water jet that is angled 75 degrees or more relative to the longitudinal axis of the pipe.

In one embodiment, the pipe is provided with a plurality of holes provided along the outer periphery of a front portion of the pipe. Hereby, it is possible to inject water radially through the holes.

In practical embodiments, a water pump is flow connected to a water supply and pumping water through the water conduit to the front end of the pipe for loosening sand in the bottom region of the slurry tank. Advantageously, the water supply is different from the slurry tank so that clean water is supplied and clogging is avoided.

A suction unit is flow-connected to the suction duct and configured for providing suction force in the suction duct for receiving the sand from the tank and, thus, removing the loosened sand from the bottom region by suction through the suction duct after the sand has been stirred-up by the injected water.

For example, at the front end of the pipe, the suction duct is located centrally and the water conduit peripherally around the suction duct. Water is expelled at the front end of the pipe peripherally around the suction area so that the sand is efficiently loosened and stirred up right in front of the suction duct, facilitating the watered sand to be sucked out of the bottom region of the tank.

Optionally, the water conduit is arranged for expelling by a water jet largely in the form of a hollow cylinder or hollow expanding cone around the suction duct. In order to achieve such hollow conical flow, flow guides are provided in or at the water duct at the front end of the pipe.

The method and system have proven to be especially useful for removal of sand from the bottom region of biological slurry tanks in biogas production plants.

SHORT DESCRIPTION OF THE DRAWINGS

The invention will be explained in more detail with reference to the drawing, where

FIG. 1 illustrates a slurry tank as used in biogas production plants;
FIG. 2 A) shows an access port with a closed valve in greater detail; B) shows an access port with an open valve in greater detail;
FIG. 3 A) shows a pipe guide mounted to the access port; B) illustrates a pipe through the pipe guide;
FIG. 4 shows a preferred embodiment of a pipe;
FIG. 5 shows the pipe of FIG. 4 inserted into the tank;
FIG. 6 is an overview sketch of the system;
FIG. 7 illustrates an example of a pipe in A) perspective view and B) front view, and C) in a cross-sectional side view with indications for the flow;
FIG. 8 shows a cross-sectional view of a pipe that basically corresponds to the one shown in Fig. 7C;
FIG. 9 shows a pipe provided with a cutter head;
FIG. 10 shows another pipe provided with a cutter head;
FIG. 11 shows a pipe provided with a plurality of nozzles arranged to inject water;
FIG. 12 shows a cross-sectional view of a pipe provided with flow guides;
FIG. 13 shows a cross-sectional view of another pipe provided with flow guides;
FIG. 14 shows a cross-sectional view of a further pipe provided with flow guides;
FIG. 15 shows a cross-sectional view of a pipe provided with nozzles and
FIG. 16 shows a perspective view of a pipe provided with holes.

DETAILED DESCRIPTION / PREFERRED EMBODIMENT

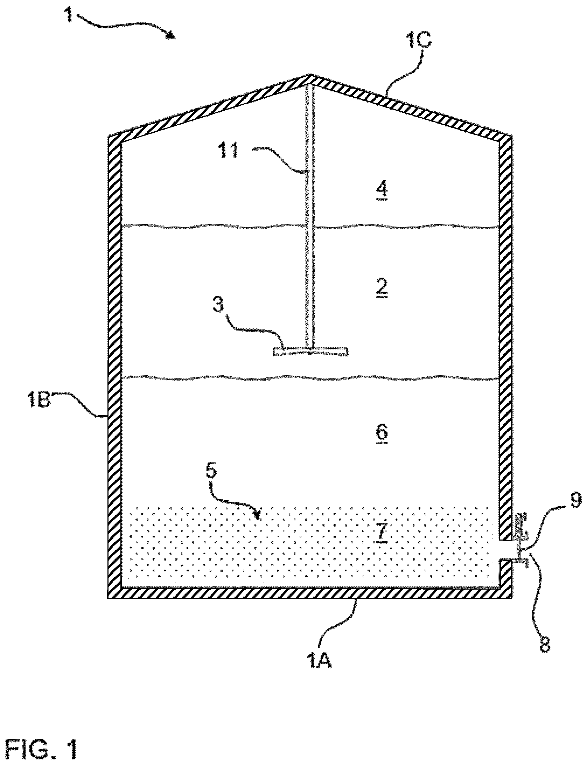

FIG. 1 shows a slurry tank 1 having a bottom 1A, side walls 1B, and a roof 1C. Inside the tank 1, slurry 2 is stored and stirred by a stirrer 3 on a driving shaft 11 for enhancing efficiency for production of biogas, which accumulates in the top region 4 under the roof 1C. In the bottom region 5 of the bioreactor 6, sediments 7 accumulate, in particular sand, which recently has become common practice as a floor covering for the animals in animal husbandry. In order to easily access the bottom region 5 from outside the tank 1, the tank is provided with an access port 8, which during normal operation is closed by a valve 9.

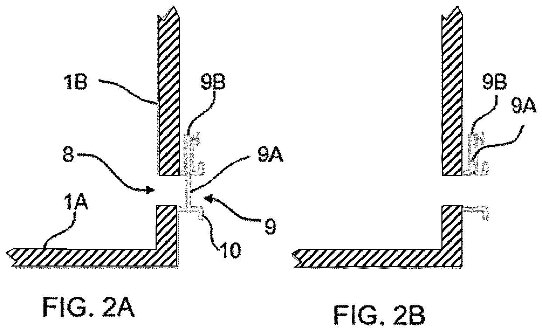

FIG. 2A shows the access port 8 in greater detail in closed state and FIG. 2B in open state. The valve 9 comprises a valve plate 9A that slides in a slot 9B for opening or closing, respectively, the access port 8. This type of valve is only shown as an illustrative example, and other valve types are possible to use.

The access port 8 is provided with a flange 10 to which pipe flanges or pipe guides can be mounted. For example, the flange 10 is circular around a circular access port 8, although this is not necessary for the invention. For ease of mounting, the flange 10 is optionally provided with a plurality of bore holes through the flange 10 for receiving bolts when pipe equipment is fastened to the flange 10.

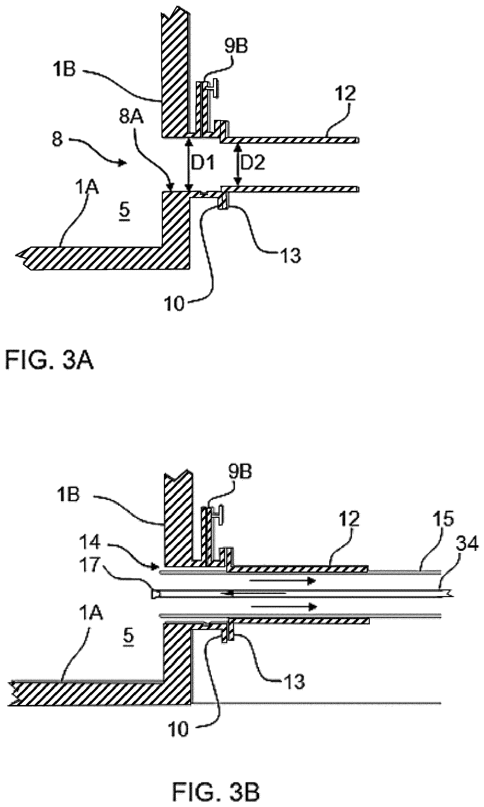

FIG. 3A shows a rigid pipe guide 12 that has a connecting flange 13 at its end, which dimensionally is cooperating with the flange 10 of the access port 8, for example having a matching plurality of bore holes for fastening the flanges 10 and 13 to each other.

The access port 8 has an inner free diameter D1. The term "free" diameter should be understood as the largest diameter of a circular cylindrical pipe that fits through the access port 8. This terminology is used because the access port 8 is not necessarily circular, or the flange 10 of the access port may have a different diameter than the rest of the access port, or there may be other types of obstacles along the inner rim of the access port 8.

The inner diameter D2 of the of the pipe guide 12 is smaller than the inner free diameter D1. For example, the inner diameter D2 is at least 5%, optionally at least 10%, smaller than D1. This implies that a pipe 15 that fits through the pipe guide 12 is guaranteed to also fit through the access port 8, even if the access port 8 is corroded or has biological debris on its inner wall 8A or other types of obstacles along the rim. Typically, the pipe guide 12 is rigid for giving a good support and directional guidance to the pipe 15.

For operation, the flange 13 of the pipe guide 12 is mounted onto the flange 10 for fixing the pipe guide 12 to the access port 8, and a pipe 15 is inserted through the pipe guide 12 and through the access port 8 into the bottom region 5 of the tank 1.

FIG. 3B illustrates a pipe 15 that has been inserted through the pipe guide 12 and into the bottom region 5 of the tank 1. The diameter of the pipe 15 is provided such that the pipe 15 fits tightly into the pipe guide 12, preventing leaks, but still allowing the pipe to slide through the pipe guide 12. Sealings, typically polymer seals, such as O-rings, between the pipe guide 12 and the pipe 15 are not illustrated but can be provided as an option. Such seals also may accommodate tolerances between the inner wall of the pipe guide 12 and the pipe 15.

There is no problem of corrosion and debris that potentially could prevent proper sliding of the pipe 15 in the pipe guide 12, as the pipe guide 12 and the pipe 15 are cleaned after each use, seeing that the pipe guide 12, typically, is demounted again from the flange 10 of the access port 8. Even in the case that the pipe guide 12 is not demounted, it is easy to clean it by water flushing after removal of the pipe 15 from the pipe guide 12.

As the outer diameter of the pipe 15 is approximately equal to the inner diameter D2 of the pipe guide 12 and, thus, smaller than the inner free diameter D1 of the access port 8, there is a gap 14 between the pipe 15 and the inner wall 8A of the access port 8, which allows free and easy insertion of the pipe 15.

In some embodiments, there is provided an inner tube 34 inside the pipe 15 for injecting water into the bottom region 5 from the front end of the pipe 15. Optionally, the inner tube 34 has a nozzle 17 for controlled direction of the water.

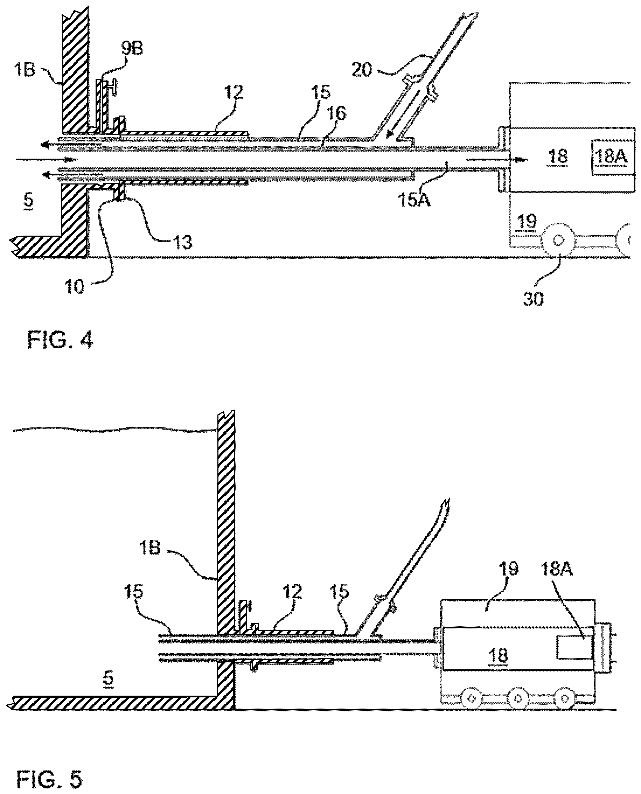

FIG. 4 illustrates a preferred embodiment in which the pipe 15 comprises a central suction duct 15A, which is used for vacuum suction of the sand and other particulate matter from the bottom region 5 of the tank 1 into a suction unit 18 as part of a vehicle 19. The pipe 15 comprises a peripheral water conduit 16 which is used for injection of water in to the bottom region 5 of the tank 1, the water coming through a water supply tube 20 from an external water tank. The external water tank is in contrast to the disclosure of the aforementioned CA02315670, where water is taken from the slurry tank. By using an external water supply, it is made sure that the liquid through the water tube used is actually water and the water pump is not clogged by biological debris.

FIG. 5 exemplifies the vehicle 19 as a trailer to which the pipe 15 is mounted. Alternatively, the vehicle 19 could be a truck. The vehicle 19 is on wheels 30 and can be moved. In the shown configuration, the pipe 15 is mounted directly to the vehicle 19. However, in typical configurations used in practice, there would advantageously be provided a flexible tube as a transition between the pipe 15 and the vehicle 19 in order make the movement more flexible. In such case, the pipe 15 is advantageously supported by a support that is provided with wheels 30 for rolling on the ground.

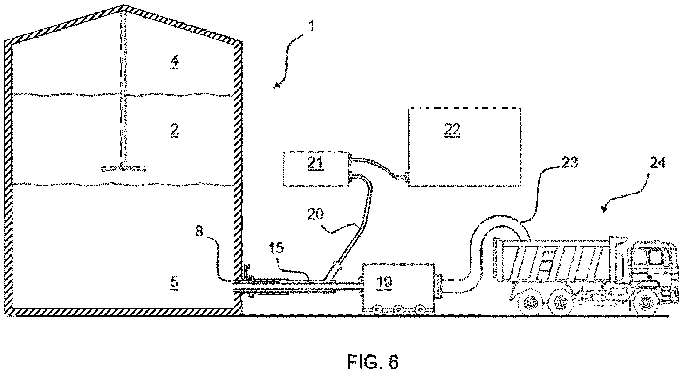

FIG. 6 illustrates an embodiment, where the vehicle 19 in the form of a trailer is connected by a discard pipe 23 to a transport lorry 24, which collects the sand and is used for transporting it away from the site. Water is provided from an external water tank 22, separate from the vehicle 19 and separate from the lorry 24. A pump 21 delivers the proper pressure for the flushing through the pipe 15. By providing the vehicle 19 with the suction pump, one lorry 24 after the other can be filled without changing the overall configuration of the system. The use of multiple lorries 24, one after the other or several simultaneously, is useful due to the large size of biological tanks 1 in biogas production plants. By providing the water pump 21 and vehicle 19 with the suction pump 18A separately from the lorries 24, a larger flexibility is given than if the water tank 22 and water pump 21 as well as suction pump 18A is part of the lorry 24.

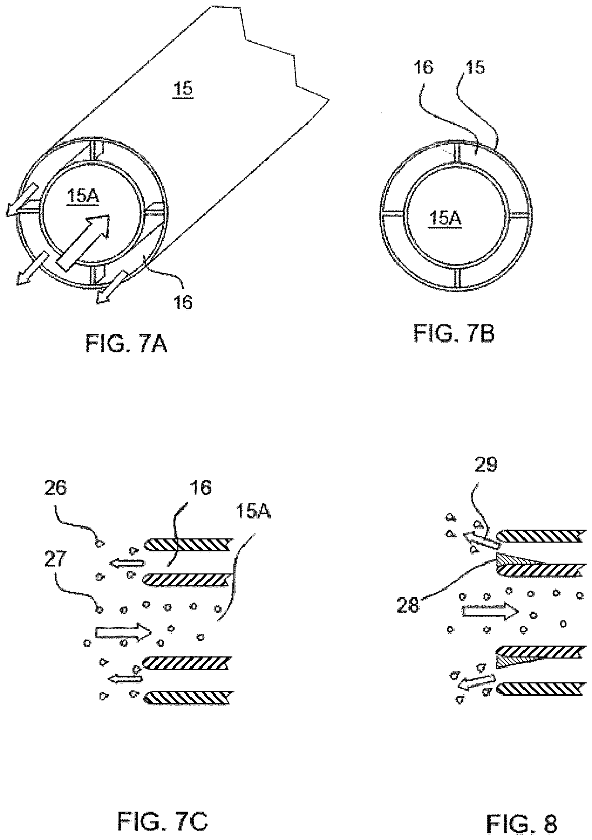

FIG. 7A and FIG. 7B illustrate an embodiment in which the pipe 15 comprises a central suction duct 15A for the suction removal of the sand and a peripheral water conduit 16 for supplying water to the front end of the suction duct 15A. The water conduit 16 is arranged peripherally around the central suction duct 15A and delimited by the outer wall of the central duct 15A and the inner wall of the pipe 15, forming a hollow cylinder. Stabilizers, exemplified as ribs between the suction duct 15A and the inner wall of the pipe 15 maintains the stability of the arrangement with the suction duct 15A. Flow directions are indicated with arrows in FIG. 7A. This embodiment is also indicated as an example for a pipe in the drawings in FIG. 4, 5, and 6.

FIG. 7C illustrates flow of water 26 out of the water conduit 16 and the suction of sand 27 into the central suction duct 15A.

As illustrated with the embodiment of FIG. 8, flow guides 28 are optionally used for directing the water flow direction 29 from the water conduit 16 at the end of the pipe 15, for example an inclined outward-directed flow, the direction of which has a radial component outwards away from a central axis of the pipe 15.

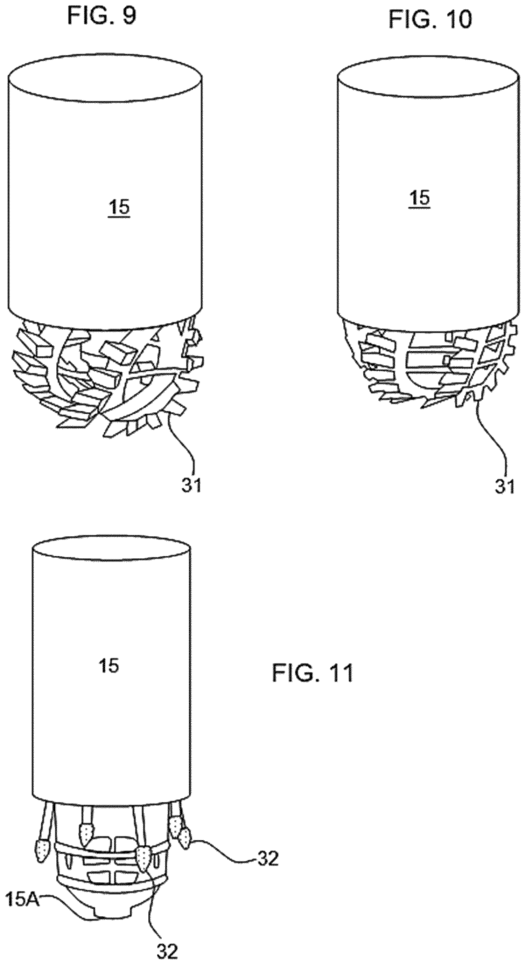

FIG. 9 illustrates the front portion of a pipe 15 provided with a cutter head 31. FIG. 10 show a front portion of another pipe provided with a cutter head. The cutter head 31 is provided in the distal end of the pipe 15. Accordingly, the cutter head 31 is arranged and configured to be used to dredge sediments in the bottom region of a bioreactor. The cutter head 31 comprises a plurality of protruding teeth arranged at the periphery of the cutter head 31. In one embodiment, the teeth are integrated in the periphery of the cutter head 31. In one embodiment, the cutter head 31 is equipped with receiving structures configured to detachably attach replaceable teeth. The latter embodiment is designed for easily changing the teeth after they have worn out. The width of the pipe 15 is equal to or exceeds the width of the cutter head 31, hereby, it is possible to insert the pipe 15 into the pipe guide.

In accordance with the invention, there is provided an inner tube inside the pipe 15 for injecting water into the bottom region from the distal end (front end) of the pipe 15.

FIG. 11 illustrates a front portion of a pipe 15 having a head portion provided with a plurality of nozzles 32 arranged to inject water. The nozzles 32 are configured to enhance the water injection capacity of the pipe 15. The pipe 15 comprises a central suction duct 15A, which is used for vacuum suction of the sand and other particulate matter from the bottom region of the tank. In one embodiment, the pipe 15 comprises a central suction duct 15A. In one embodiment, there is provided an inner tube inside the pipe 15 for injecting water into the bottom region from the distal end of the pipe 15.

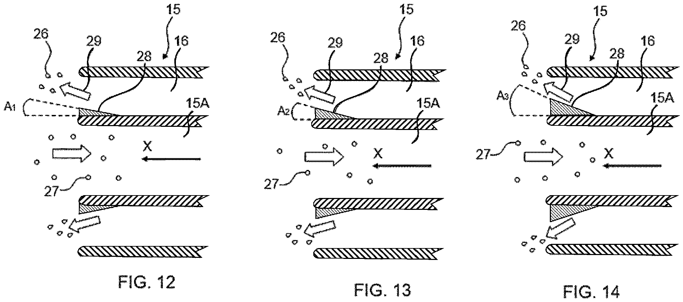

FIG. 12 illustrates a cross-sectional view of a pipe provided with a flow guide 28 arranged and configured to angle the injected water 26 relative to the longitudinal axis X of the pipe 15. The flow guide 28 is arranged to angle the injected water 26 with a predefined angle $A_1$ relative to the longitudinal axis X of the pipe 15. The angle $A_1$ corresponds to the water flow direction 29 that is indicated. The water 26 flowing through the water conduit 16 will initially have a flow direction parallel to the longitudinal axis X of the pipe 15. The flow guide 28 will, however, change the water flow direction 29 as indicated. The pipe 15 is configured to suck sand 27 or other types of solid into the central suction duct 15A.

FIG. 13 and FIG. 14 illustrate cross-sectional view of other pipes 15 provided with flow guides. These pipes 15 basically correspond to the one shown in and explained with reference to FIG. 12. The flow guides 28, however, are angled slightly differently as in

FIG. 13 the flow guide 28 is arranged to angle the injected water 26 with a predefined angle $A_2$ relative to the longitudinal axis X of the pipe 15. In FIG. 14 the flow guide 28 is arranged to angle the injected water 26 with a predefined angle $A_3$ relative to the longitudinal axis X of the pipe 15. It can be seen that $A_1$ is smaller than $A_2$ which is smaller than $A_3$. This can be expressed in the following way:

$$(1) \quad A_1 < A_2 < A_3$$

In one embodiment, the angles $A_1$, $A_2$, $A_3$ are in the range 10-60 degrees.

In one embodiment, the angles $A_1$, $A_2$, $A_3$ are in the range 25-50 degrees.

In one embodiment, the angles $A_1$, $A_2$, $A_3$ are in the range 20-45 degrees.

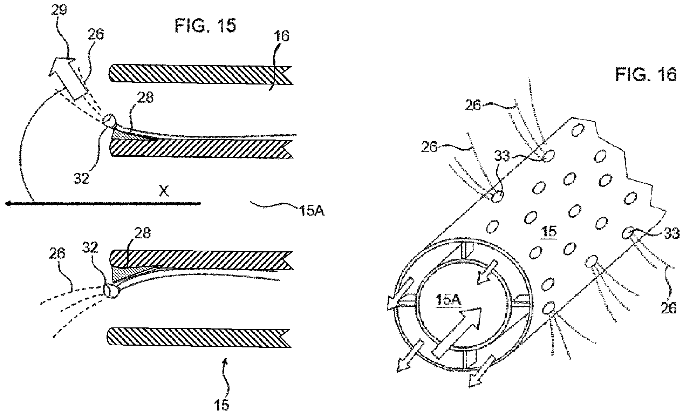

FIG. 15 illustrates a cross-sectional view of a pipe 15 provided with nozzles 32. The pipe 15 basically corresponds to the one shown in FIG 12. The nozzles 32 are, however, added. In one embodiment, each nozzle 32 is a rotatably mounted rotation nozzle 32, wherein the water flow through the nozzle 32 creates rotation of the nozzle 32. The nozzle 32 is provided at the end distal end of a water supply pipe or hose 33.

The average water flow direction 29 of the water 26 injected by the nozzle 32 is indicated.

In one embodiment, the nozzles 32 are arranged and configured to inject a water jet that is angled more than 45 degrees relative to the longitudinal axis X of the pipe 15.

In one embodiment, the nozzles 32 are arranged and configured to inject a water jet that is angled more than 60 degrees relative to the longitudinal axis X of the pipe 15.

In one embodiment, the nozzles 32 are arranged and configured to inject a water jet that is angled 75 degrees or more relative to the longitudinal axis X of the pipe 15.

FIG. 16 illustrates a perspective view of the front portion of a pipe 15. The pipe 15 is provided with holes 33. The holes 33 are provided along the outer periphery of the front portion of the pipe 15. It can be seen that water 26 is radially injected through the holes 33.

There is provided an inner tube (not shown) inside the pipe 15 for injecting water 26. The water 26 is, however, also injected out through the holes 33. The pipe 15 comprises a central suction duct 15A, which is used for vacuum suction of the sand and other particulate matter.

**Reference numbers**

1 tank
2 slurry
3 stirrer
4 top region
5 bottom region
6 bioreactor
7 sediments
8 access port
8A inner wall of access port
9 valve
9A valve plate
9B slot for valve plate 9A
10 flange
11 driving shaft of stirrer
12 pipe guide
13 flange
14 gap between inner wall 8A of acces port and pipe 15
15 pipe
15A suction duct in pipe 15
16 water conduit
17 nozzle
18 suction unit
18A suction pump of suction unit 18
19 vehicle
20 water supply tube
21 water pump
22 water tank
23 discard pipe
24 transport lorry
26 water
27 sand
28 flow guides
29 water flow direction
30 wheel
31 cutter head
32 nozzle
33 hole
34 inner tube
$A_1$ angle
$A_2$ angle
$A_3$ angle
X longitudinal axis

**Claims**

1. A method of removing sand from a biological slurry tank (1), the slurry tank (1) comprising access port (8) at a bottom region (5) of the tank (1) for access from outside the tank (1) to the bottom region (5), the access port (8) comprising a valve (9) for closing and opening the access port (8), the access port (8) having a free inner diameter D1, which is the largest diameter of a circular cylindrical pipe fitting through the access port (18);
the method comprising:

   - providing a pipe guide (12) and attaching an end of the pipe guide (12) to the access port (8) by a liquid tight connection, the pipe guide (12) having a cylindrical inner wall with an inner pipe guide diameter D2;
   - providing a pipe (15) slidingly in the pipe guide (12) with liquid-tight sealing between the pipe (15) and the pipe guide (12) to prevent leakage between the pipe (15) and the pipe guide (12); the pipe (15) further comprising a water conduit (16) inside the pipe (15) for transport of water (26) through the pipe (15) and a suction duct (15A) inside the pipe (15), separate from the water conduit (16), for transport of sand through the pipe (15) to a suction unit (18);
   - moving a front end of the pipe (15) through the access port (8) into the bottom region (5) of the tank (1) by sliding the pipe (15) inside and along the pipe guide (12);
   - pumping water (26) through the water conduit (16) to the inserted front end of the pipe (15) for loosening sand in the bottom region (5) of the slurry tank (1) and removing the loosened sand from the bottom region (5) by suction through the suction duct (15A), wherein the pipe guide diameter D2 is smaller than the free inner diameter D1 of the access port (8) for unhindered motion of the pipe (15) through the access port (8), and the method comprises expelling water (26) at the front end of the pipe (15) around the suction area and by suction pumping the watered sand into the suction duct (15A), **characterised in that** at the front end of the pipe (15), the suction duct (15A) is located centrally and the water conduit (16) peripherally around the suction duct (15A), wherein the pipe (15) has a longitudinal axis (X), wherein the pipe (15) is provided with a flow guide (28) arranged and configured to angle the injected water relative to the longitudinal axis (X) of the pipe (15).

2. Method according to claim 1, wherein the diameter of the suction duct (15A) is at least 5% smaller than the free inner diameter D1 of the access port (8).

3. Method according to any preceding claim, wherein the suction unit (18) has an inlet port connected to the rear end of a suction duct (15A) and has an outlet port connected to a first end of a discard pipe (23) and a suction pump (18A) in between the inlet port and the outlet port; wherein the second end of the discard pipe (23) is flow-connected to a lorry (24) separate from the suction unit (18); wherein the method comprises pumping the sand by the suction pump (18A) from the bottom region (5) of the tank (1) through the suction duct (15A) by suction and pumping it further through the discard pipe (23) into multiple lorries (24), simultaneously or one after the other.

4. A system for removing sand from a biological slurry tank (1) by a method according to any preceding claim, the slurry tank (1) comprising an access port (8) at a bottom region (5) of the tank (1) for access from outside the tank (1) to the bottom region (5), the access port (8) comprising a valve (9) for closing and opening the access port (8), the access port (8) having a free inner diameter D1, which is the largest diameter of a pipe (15) fitting through the access port (8); the system comprising

- a water pump (21) flow connected to a water supply (22);
- a pipe guide (12) having a connector (13) at its one end for attachment of the pipe guide (12) to the access port (8) by a liquid tight connection, the pipe guide (12) having an inner cylindrical wall with an inner pipe guide diameter D2;
- a pipe arranged slidingly in the pipe guide (12) with liquid-tight sealing between the pipe (15) and the pipe guide (12) to prevent leakage between the pipe and the pipe guide (12); the pipe (15) further comprising a water conduit (16) inside the pipe (15) and flow-connected to the water pump for transport of water (26) through the pipe (15); the pipe (15) comprising a suction duct (15A) inside the pipe (15), separate from the water conduit (16), for transport of sand through the pipe (15);
- a suction unit (18) flow connected to the suction duct (15A) and configured for providing suction force in the suction duct (15A) and for receiving the sand from the tank (1); wherein, the inner pipe guide diameter D2 is smaller than the free inner diameter D1 of the access port (8) for unhindered motion of the pipe (15) through the access port (8), wherein the water pump (21) is configured to expel water (26) at the front end of the pipe (15) around the suction area and the suction unit (18) is configured to suction pump the watered sand into the suction duct (15A),

**characterised in that** at the front end of the pipe (15), the suction duct (15A) is located centrally and the water conduit (16) peripherally around the suction duct (15A), wherein the pipe (15) has a longitudinal axis (X), wherein the pipe (15) is provided with a flow guide (28) arranged and configured to angle the injected water relative to the longitudinal axis (X) of the pipe (15).

5. System according to claim 4, wherein the diameter of the suction duct (15A) is at least 5% smaller than the free inner diameter D1 of the access port (8).

6. System according to claim 4 or 5, wherein the water supply (22) is separate from the slurry tank (1).

7. System according to claim 4, wherein the suction unit (18) has an inlet port connected to the rear end of the suction duct (15A) and has an outlet port connected to a first end of a discard pipe (23) and a suction pump (18A) in between the inlet port and the outlet port for pumping the sand (27) through the suction duct (15A) by suction and pumping it into the discard pipe (23) for further transport of the sand (27); wherein the second end of the discard pipe (23) is flow-connected to a lorry (24) for receiving the sand (27), wherein the lorry (24) is separate from the suction unit (28) in order for the suction unit (18) to fill the sand (27) into multiple lorries (24), simultaneously or one after the other.

8. System according to anyone of the claims 4-7, wherein, at the front end of the pipe (15), the suction duct (15A) is located centrally and the water conduit (16) peripherally around the suction duct (15A) for providing water (26) at the front end of the pipe (15) around a suction area of the suction duct (15A).

9. System according to claim 8, wherein water flow guides (28) are provided in the water conduit (16) at the front end of the pipe (15) for guiding water in an inclined outward direction relatively to a central axis of the pipe (16).

10. Use of a method according to anyone of the claims 1-3 or of a system according to any one of the claims 4-9 for removing sand (27) from a biological slurry tank in a biogas production plant.

**Patentansprüche**

1. Verfahren zum Entfernen von Sand aus einem biologischen Schlammbehälter (1), wobei der Schlammbehälter (1) eine Zugangsöffnung (8) an einem Bodenbereich (5) des Behälters (1) für den Zugang von außerhalb des Behälters (1) zu dem Bodenbereich (5) umfasst, wobei die Zugangsöffnung (8) ein Ventil (9) zum Schließen und Öffnen der Zugangsöffnung (8) umfasst, wobei die Zugangsöffnung (8) einen freien Innendurchmesser D1 aufweist, der der größte Durchmesser eines kreiszylindrischen Rohrs ist, das durch die Zugangsöffnung (18) passt;
wobei das Verfahren Folgendes umfasst:

- Bereitstellen einer Rohrführung (12) und Anbringen eines Endes der Rohrführung (12) an der Zugangsöffnung (8) durch eine flüssigkeitsdichte Verbindung, wobei die Rohrführung (12) eine zylindrische Innenwand mit einem inneren Rohrführungsdurchmesser D2 aufweist;
- Bereitstellen eines Rohrs (15), das in der Rohrführung (12) gleitet, mit einer flüssigkeitsdichten Abdichtung zwischen dem Rohr (15) und der Rohrführung (12), um eine Leckage zwischen dem Rohr (15) und der Rohrführung (12) zu verhindern; wobei das Rohr (15) ferner eine Wasserleitung (16) innerhalb des Rohrs (15) für den Transport von Wasser (26) durch das Rohr (15) und eine Saugleitung (15A) innerhalb des Rohrs (15), die von der Wasserleitung (16) getrennt ist, für den Transport von Sand durch das Rohr (15) zu einer Saugeinheit (18) umfasst;
- Bewegen eines vorderen Endes des Rohrs (15) durch die Zugangsöffnung (8) in den Bodenbereich (5) des Behälters (1) durch Verschieben des Rohrs (15) innerhalb und entlang der Rohrführung (12);
- Pumpen von Wasser (26) durch die Wasserleitung (16) zum eingeführten vorderen Ende des Rohrs (15), um Sand im Bodenbereich (5) des Schlammbehälters (1) aufzulockern, und Entfernen des aufgelockerten Sandes aus dem Bodenbereich (5) durch Saugen durch die Saugleitung (15A), wobei der Rohrführungsdurchmesser D2 kleiner ist als der freie Innendurchmesser D1 der Zugangsöffnung (8), um eine ungehinderte Bewegung des Rohrs (15) durch die Zugangsöffnung (8) zu ermöglichen, und wobei das Verfahren das Ausstoßen von Wasser (26) am vorderen Ende des Rohrs (15) um den Ansaugbereich herum und durch Saugpumpen des gewässerten Sandes in den Saugkanal (15A) umfasst, **dadurch gekennzeichnet, dass** am vorderen Ende des Rohrs (15) der Saugkanal (15A) mittig und die Wasserleitung (16) in Umfangsrichtung um den Saugkanal (15A) herum angeordnet ist, wobei das Rohr (15) eine Längsachse (X) aufweist, wobei das Rohr (15) mit einer Strömungsführung (28) vorgesehen ist, die so angeordnet und konfiguriert ist, dass sie das eingespritzte Wasser relativ zur Längsachse (X) des Rohrs (15) abwinkelt.

2. Verfahren nach Anspruch 1, wobei der Durchmesser des Saugkanals (15A) mindestens 5 % kleiner ist als der freie Innendurchmesser D1 der Zugangsöffnung (8).

3. Verfahren nach einem der vorhergehenden Ansprüche, wobei die Saugeinheit (18) eine Einlassöffnung aufweist, die mit dem hinteren Ende eines Saugkanals (15A) verbunden ist, und eine Auslassöffnung aufweist, die mit einem ersten Ende eines Auswurfrohrs (23) verbunden ist, und eine Saugpumpe (18A) zwischen der Einlassöffnung und der Auslassöffnung; wobei das zweite Ende des Auswurfrohrs (23) mit einem von der Saugeinheit (18) getrennten Lastwagen (24) strömungsverbunden ist; wobei das Verfahren das Pumpen des Sandes durch die Saugpumpe (18A) vom Bodenbereich (5) des Behälters (1) durch die Saugleitung (15A) durch Saugen und Weiterpumpen durch das Auswurfrohr (23) in mehrere Lastwagen (24) gleichzeitig oder nacheinander umfasst.

4. System zum Entfernen von Sand aus einem biologischen Schlammbehälter (1) durch ein Verfahren nach einem der vorhergehenden Ansprüche, wobei der Schlammbehälter (1) eine Zugangsöffnung (8) an einem Bodenbereich (5) des Behälters (1) für den Zugang von außerhalb des Behälters (1) zu dem Bodenbereich (5) umfasst, wobei die Zugangsöffnung (8) ein Ventil (9) zum Schließen und Öffnen der Zugangsöffnung (8) umfasst, wobei die Zugangsöffnung (8) einen freien Innendurchmesser D1 aufweist, der der größte Durchmesser eines Rohrs (15) ist, das durch die Zugangsöffnung (8) passt;

wobei das System Folgendes umfasst:

- eine Wasserpumpe (21), die mit einer Wasserversorgung (22) strömungsverbunden ist;
- eine Rohrführung (12), die an ihrem einen Ende ein Verbindungsstück (13) zur Befestigung der Rohrführung (12) an der Zugangsöffnung (8) durch eine flüssigkeitsdichte Verbindung aufweist, wobei die Rohrführung (12) eine innere zylindrische Wand mit einem inneren Rohrführungsdurchmesser D2 aufweist;
- ein Rohr, das gleitend in der Rohrführung (12) angeordnet ist, mit einer flüssigkeitsdichten Abdichtung zwischen dem Rohr (15) und der Rohrführung (12), um eine Leckage zwischen dem Rohr und der Rohrführung (12) zu verhindern; wobei das Rohr (15) ferner eine Wasserleitung (16) innerhalb des Rohrs (15) und in Strömungsverbindung mit der Wasserpumpe zum Transport von Wasser (26) durch das Rohr (15) umfasst; wobei das Rohr (15) einen Saugkanal (15A) innerhalb des Rohrs (15) umfasst, der von der Wasserleitung (16) getrennt ist, um Sand durch das Rohr (15) zu transportieren;
- eine Saugeinheit (18), die mit der Saugleitung (15A) strömungsverbunden und konfiguriert ist, um eine Saugkraft in der Saugleitung (15A) bereitzustellen und den Sand aus dem Behälter (1) aufzunehmen;

wobei der innere Rohrführungsdurchmesser D2 kleiner ist als der freie Innendurchmesser D1 der Zugangsöffnung (8), um eine ungehinderte Bewegung des Rohrs (15) durch die Zugangsöffnung (8) zu ermöglichen,
wobei die Wasserpumpe (21) konfiguriert ist, um Wasser (26) am vorderen Ende des Rohrs (15) um den Ansaugbereich herum auszustoßen, und die Saugeinheit (18) konfiguriert ist zum Saugpumpen des gewässerten Sands in den Saugkanal (15A), **dadurch gekennzeichnet, dass** am vorderen Ende des Rohrs (15) der Saugkanal (15A) zentral und die Wasserleitung (16) in Umfangsrichtung um den Saugkanal (15A) herum angeordnet ist, wobei das Rohr (15) eine Längsachse (X) aufweist, wobei das Rohr (15) mit einer Strömungsführung (28) vorgesehen ist, die so angeordnet und konfiguriert ist, dass sie das eingespritzte Wasser relativ zur Längsachse (X) des Rohrs (15) anwinkelt.

5. System nach Anspruch 4, wobei der Durchmesser des Saugkanals (15A) mindestens 5 % kleiner ist als der freie Innendurchmesser D1 der Zugangsöffnung (8).

6. System nach Anspruch 4 oder 5, wobei die Wasserversorgung (22) vom Schlammbehälter (1) getrennt ist.

7. System nach Anspruch 4, wobei die Saugeinheit (18) eine Einlassöffnung aufweist, die mit dem hinteren Ende des Saugkanals (15A) verbunden ist, und eine Auslassöffnung aufweist, die mit einem ersten Ende eines Auswurfrohrs (23) verbunden ist, und eine Saugpumpe (18A) zwischen der Einlassöffnung und der Auslassöffnung, um den Sand (27) durch Saugen durch den Saugkanal (15A) zu pumpen und ihn in das Auswurfrohr (23) zum weiteren Transport des Sandes (27) zu pumpen; wobei das zweite Ende des Auswurfrohrs (23) mit einem Lastwagen (24) zur Aufnahme des Sandes (27) strömungsverbunden ist, wobei der Lastwagen (24) von der Saugeinheit (28) getrennt ist, damit die Saugeinheit (18) den Sand (27) in mehrere Lastwagen (24) gleichzeitig oder nacheinander füllen kann.

8. System nach einem der Ansprüche 4 bis 7, wobei am vorderen Ende des Rohrs (15) der Saugkanal (15A) zentral und die Wasserleitung (16) in Umfangsrichtung um den Saugkanal (15A) herum angeordnet ist, um Wasser (26) am vorderen Ende des Rohrs (15) um einen Ansaugbereich des Saugkanals (15A) herum bereitzustellen.

9. System nach Anspruch 8, wobei in der Wasserleitung (16) am vorderen Ende des Rohrs (15) Wasserströmungsführungen (28) vorgesehen sind, um Wasser in einer geneigten Auswärtsrichtung relativ zu einer Mittelachse des Rohrs (16) zu führen.

10. Verwendung eines Verfahrens nach einem der Ansprüche 1 bis 3 oder eines Systems nach einem der Ansprüche 4 bis 9 zum Entfernen von Sand (27) aus einem biologischen Schlammbehälter in einer Biogaserzeugungsanlage.

**Revendications**

1. Procédé pour éliminer du sable à partir d'un réservoir de boue biologique (1), le réservoir de boue (1) comprenant un orifice d'accès (8) dans une région inférieure (5) du réservoir (1), pour l'accès, depuis l'extérieur du réservoir (1), à la région inférieure (5), l'orifice d'accès (8) comprenant une vanne (9) pour fermer et ouvrir l'orifice d'accès (8), l'orifice d'accès (8) ayant un diamètre intérieur libre D1, qui est le plus grand diamètre d'un tuyau cylindrique circulaire s'ajustant à travers l'orifice d'accès (18) ;
le procédé comprenant :

- la fourniture d'un guide de tuyau (12) et la fixation d'une extrémité du guide de tuyau (12) à l'orifice d'accès (8) par le biais d'un raccordement hermétique aux liquides, le guide de tuyau (12) ayant une paroi intérieure cylindrique avec un diamètre intérieur de guide de tuyau D2 ;
- la fourniture d'un tuyau (15), de façon coulissante dans le guide de tuyau (12), avec une étanchéité hermétique aux liquides entre le tuyau (15) et le guide de tuyau (12), pour empêcher des fuites entre le tuyau (15) et le guide de tuyau (12) ; le tuyau (15) comprenant en outre un conduit d'eau (16), à l'intérieur du tuyau (15), pour le transport d'eau (26) à travers le tuyau (15), et une conduite d'aspiration (15A), à l'intérieur du tuyau (15), séparée du conduit d'eau (16), pour le transport de sable à travers le tuyau (15) jusqu'à une unité d'aspiration (18) ;
- le déplacement d'une extrémité avant du tuyau (15), à travers l'orifice d'accès (8), dans la région inférieure (5) du réservoir (1), en faisant coulisser le tuyau (15) à l'intérieur et le long du guide de tuyau (12) ;
- le pompage d'eau (26), à travers le conduit d'eau (16), jusqu'à l'extrémité avant insérée du tuyau (15), pour ameublir du sable dans la région inférieure (5) du réservoir de boue (1) et éliminer le sable ameubli, à partir de la région inférieure (5), par aspiration à travers la conduite d'aspiration (15A), dans lequel le diamètre de guide de tuyau D2 est inférieur au diamètre intérieur libre D1 de l'orifice d'accès (8) pour le déplacement libre du tuyau (15) à travers l'orifice d'accès (8), et le procédé comprend l'expulsion d'eau (26), à l'extrémité avant du tuyau (15), autour de la zone d'aspiration, et, par aspiration, le pompage du sable, fluidifié avec l'eau, dans la conduite d'aspiration (15A), **caractérisé en ce que**, à l'extrémité avant du tuyau (15), la conduite d'aspiration (15A) est située de façon centrale et le conduit d'eau (16) périphériquement autour de la conduite d'aspiration (15A), dans lequel le tuyau (15) a un axe longitudinal (X), dans lequel le tuyau (15) est pourvu d'un guide d'écoulement (28) agencé et configuré pour incliner l'eau injectée relativement à l'axe longitudinal (X) du tuyau (15).

2. Procédé selon la revendication 1, dans lequel le diamètre de la conduite d'aspiration (15A) est au moins inférieur de 5 % au diamètre intérieur libre D1 de l'orifice d'accès (8).

3. Procédé selon une quelconque revendication précédente, dans lequel l'unité d'aspiration (18) a un orifice d'entrée, raccordé à l'extrémité arrière d'une conduite d'aspiration (15A), et a un orifice de sortie, raccordé à une première extrémité d'un tuyau de rejet (23), et une pompe d'aspiration (18A) entre l'orifice d'entrée et l'orifice de sortie ; dans lequel la seconde extrémité du tuyau de rejet (23) est raccordée en écoulement à un camion (24) séparé de l'unité d'aspiration (18) ; dans lequel le procédé comprend le pompage du sable, par la pompe d'aspiration (18A), depuis la région inférieure (5) du réservoir (1), à travers la conduite d'aspiration (15A), par aspiration, et son pompage supplémentaire, à travers le tuyau de rejet (23), dans de multiples camions (24), simultanément ou les uns après les autres.

4. Système pour éliminer du sable à partir d'un réservoir de boue biologique (1), par l'intermédiaire d'un procédé selon une quelconque revendication précédente, le réservoir de boue (1) comprenant un orifice d'accès (8) dans une région inférieure (5) du réservoir (1) pour l'accès, depuis l'extérieur du réservoir (1), à la région inférieure (5), l'orifice d'accès (8) comprenant une vanne (9) pour fermer et ouvrir l'orifice d'accès (8), l'orifice d'accès (8) ayant un diamètre intérieur libre D1, qui est le plus grand diamètre d'un tuyau (15) s'ajustant à travers l'orifice d'accès (8) ;

le système comprenant

- une pompe à eau (21) raccordée en écoulement à une alimentation en eau (22) ;
- un guide de tuyau (12) ayant un raccord (13) à son extrémité pour la fixation du guide de tuyau (12) à l'orifice d'accès (8), par le biais d'un raccordement hermétique aux liquides, le guide de tuyau (12) ayant une paroi cylindrique intérieure avec un diamètre intérieur de guide de tuyau D2 ;
- un tuyau agencé de façon coulissante dans le guide de tuyau (12), avec une étanchéité hermétique aux liquides entre le tuyau (15) et le guide de tuyau (12), pour empêcher des fuites entre le tuyau et le guide de tuyau (12) ; le tuyau (15) comprenant en outre un conduit d'eau (16), à l'intérieur du tuyau (15), et raccordé en écoulement à la pompe à eau, pour le transport d'eau (26) à travers le tuyau (15) ; le tuyau (15) comprenant une conduite d'aspiration (15A), à l'intérieur du tuyau (15), séparée du conduit d'eau (16), pour le transport de sable à travers le tuyau (15) ;
- une unité d'aspiration (18) raccordée en écoulement à la conduite d'aspiration (15A) et configurée pour fournir une force d'aspiration dans la conduite d'aspiration (15A) et pour recevoir le sable à partir du réservoir (1) ;

dans lequel, le diamètre intérieur de guide de tuyau D2 est inférieur au diamètre intérieur libre D1 de l'orifice d'accès (8) pour le déplacement libre du tuyau (15) à travers l'orifice d'accès (8), dans lequel la pompe à eau (21) est configurée pour expulser de l'eau (26), à l'extrémité avant du tuyau (15), autour de la zone d'aspiration, et l'unité d'aspiration (18) est configurée pour pomper, par aspiration, le sable, fluidifié avec l'eau, dans la conduite d'aspiration (15A), **caractérisé en ce que**, à l'extrémité avant du tuyau (15), la conduite d'aspiration (15A) est située de façon centrale et le conduit d'eau (16) périphériquement autour de la conduite d'aspiration (15A), dans lequel le tuyau (15) a un axe longitudinal (X), dans lequel le tuyau (15) est pourvu d'un guide d'écoulement (28) agencé et configuré pour incliner l'eau injectée relativement à l'axe longitudinal (X) du tuyau (15).

5. Système selon la revendication 4, dans lequel le diamètre de la conduite d'aspiration (15A) est au moins inférieur de 5 % au diamètre intérieur libre D1 de l'orifice d'accès (8).

6. Système selon la revendication 4 ou 5, dans lequel l'alimentation en eau (22) est séparée du réservoir de boue (1).

**7.** Système selon la revendication 4, dans lequel l'unité d'aspiration (18) a un orifice d'entrée, raccordé à l'extrémité arrière de la conduite d'aspiration (15A), et a un orifice de sortie, raccordé à une première extrémité d'un tuyau de rejet (23), et une pompe d'aspiration (18A), entre l'orifice d'entrée et l'orifice de sortie, pour pomper le sable (27), à travers la conduite d'aspiration (15A), par aspiration, et le pomper, dans le tuyau de rejet (23), pour le transport supplémentaire du sable (27) ; dans lequel la seconde extrémité du tuyau de rejet (23) est raccordée en écoulement à un camion (24) pour recevoir le sable (27), dans lequel le camion (24) est séparé de l'unité d'aspiration (28) afin que l'unité d'aspiration (18) remplissent de multiples camions (24), simultanément ou les uns après les autres, avec le sable (27).

**8.** Système selon l'une quelconque des revendications 4 à 7, dans lequel, à l'extrémité avant du tuyau (15), la conduite d'aspiration (15A) est située de façon centrale et le conduit d'eau (16) périphériquement autour de la conduite d'aspiration (15A) pour fournir de l'eau (26), à l'extrémité avant du tuyau (15), autour d'une zone d'aspiration de la conduite d'aspiration (15A).

**9.** Système selon la revendication 8, dans lequel des guides d'écoulement d'eau (28) sont prévus dans le conduit d'eau (16), à l'extrémité avant du tuyau (15), pour guider de l'eau dans une direction inclinée vers l'extérieur relativement à un axe central du tuyau (16).

**10.** Utilisation d'un procédé selon l'une quelconque des revendications 1 à 3 ou d'un système selon l'une quelconque des revendications 4 à 9 pour éliminer du sable (27), à partir d'un réservoir de boue biologique, dans une installation de production de biogaz.

FIG. 1

FIG. 2A

FIG. 2B

FIG. 3A

FIG. 3B

FIG. 4

FIG. 5

FIG. 6

FIG. 7A

FIG. 7B

FIG. 7C

FIG. 8

FIG. 9

15

31

FIG. 10

15

31

15

FIG. 11

15A

32

32

FIG. 12

FIG. 13

FIG. 14

FIG. 15

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 02366079 C
- CA 02315670
- US 20110088732 A1
- US 2020222951 A1
- US 8734596 B1
- EP 2497578 A2